# DEMANDE DE BREVET EUROPEEN

(11) **EP 0 829 273 A1**
(43) Date de publication de la demande: **18.03.1998**
(21) Numéro de dépôt: 97460033.0
(22) Date de dépôt: 03.09.1997
(51) Int. Cl.: A61N 5/06

(54) **Installation de bronzage, dispositif et procédé de régulation automatique d'un appareil de bronzage et objet portatif apte à coopérer avec ceux-ci**

(30) Priorité: 13.09.1996 FR 9611400
(71) Demandeur: Ory, Jean-Michel, 72610 Arconnay (FR)
(72) Inventeur: Ory, Jean-Michel, 72610 Arconnay (FR)
(74) Mandataire: Vidon, Patrice

(57) **Abrégé**

L'invention concerne une installation de bronzage, un dispositif et un procédé de régulation d'un appareil de bronzage et un objet portatif apte à coopérer avec ladite installation ou ledit dispositif.

Le but de l'invention est de rendre beaucoup plus fiable les appareils de bronzage et de protéger la santé des utilisateurs.

Ce but est atteind à l'aide d'une installation (1) comprenant un appareil de bronzage (3) du type comprenant des moyens d'émission d'un rayonnement solaire artificiel et des moyens de temporisation de la durée de la séance d'irradiation, caractérisé en ce qu'elle comprend des moyens d'asservissement des moyens de temporisation et/ou des moyens d'émission à l'aide :
- de moyens de mesure de l'éclairement énergétique du rayonnement U.V.A. émis par les moyens d'émission,
- d'au moins un objet portatif comportant des données représentatives des capacités de tolérance de l'utilisateur au rayonnement solaire, et
- d'un récepteur d'au moins l'un desdits objets portatifs.

## Description

L'invention concerne une installation de bronzage, un dispositif et un procédé de régulation automatique d'un appareil de bronzage et un objet portatif apte à coopérer avec ladite installation ou ledit dispositif.

La mode du bronzage a entraîné le développement de l'implantation d'appareils de bronzage dans les salons de beauté ou les centres de remise en forme et également la création de centres de bronzage en libre-service. Ces centres sont apparus aux Etats-Unis en 1980, où leur nombre a augmenté de 300 % en 5 ans. Par ailleurs, dans les pays à moindre ensoleillement comme la Grande-Bretagne, la Hollande ou le Danemark, 10 à 15% de la population fréquentent de tels centres de bronzage au moins une fois par an.

Dans toute la suite du texte, le terme "appareil de bronzage" couvre aussi bien les cabines dans lesquelles l'utilisateur est debout que les lits dans lesquels l'utilisateur est couché ou les appareils qui permettent d'irradier seulement une partie du corps de l'utilisateur.

Les clients de ces centres sont souvent des personnes ayant des difficultés à bronzer en raison de leur peau claire et qui veulent préparer leur peau avant une exposition prolongée au soleil ou des personnes qui veulent paraître bronzées toute l'année.

Alors que l'on pensait que seuls les U.V.B pouvaient être à l'origine de cancers cutanés, des expériences effectuées en laboratoire ont permis de prouver que les U.V.A pouvaient également provoquer des lésions similaires. Ces études ont également montré que l'augmentation du risque de cancer était relatif aux expositions solaires. Sachant que la quantité d'U.V.A émise par les appareils de bronzage est cinq fois supérieure à celle émise par le soleil et que même en faible quantités des U.V.B y sont toujours associés, on comprend le danger que représentent de tels appareils lorsque l'exposition de l'utilisateur n'est pas limitée ou adaptée à ses capacités de tolérance. De plus, une exposition trop importante aux U.V. entraîne un vieillissement cellulaire, des brûlures graves, l'apparition de taches inesthétiques sur la peau ou des maladies de l'immunité.

Toutefois, pour obtenir l'effet de bronzage recherché, il faut que l'utilisateur reçoive une certaine quantité de rayons U.V. Il est donc nécessaire de réguler au mieux l'exposition afin de satisfaire l'utilisateur et de respecter sa santé.

Afin de limiter l'exposition de l'utilisateur au rayonnement, la plupart des appareils existants sont munis d'une minuterie qui interrompt leur fonctionnement au bout d'un temps d'exposition donné, calculé en fonction d'une valeur moyenne de la puissance théorique (valeur donnée par le constructeur), émise par les lampes ou tubes à U.V.

Toutefois, ces appareils ne tiennent pas compte de l'état d'usure des tubes qui ont une durée de vie de 500 heures d'émission environ. Au bout d'un certain temps d'utilisation, l'éclairement énergétique (c'est à dire le quotient de la puissance émise sous forme de rayonnement par la surface irradié), diminue. Au contraire, lorsque l'un des tubes cesse de fonctionner, il est remplacé par un nouveau tube et l'éclairement énergétique réellement délivré par l'appareil peut alors être beaucoup plus important qu'auparavant, sans que l'appareil n'ait pour autant été étalonné de nouveau. En conséquence, à temps d'exposition identique, le rayonnement reçu par l'utilisateur peut être plus important et donc plus dangereux ou plus faible et insuffisant pour obtenir l'effet de bronzage recherché.

Un autre dispositif de l'art antérieur gère le temps d'exposition en fonction d'une usure théorique des tubes, en considérant que la puissance délivrée par ces tubes décroît de façon linéaire dans le temps et en augmentant en conséquence le temps d'exposition. Or ceci n'est pas toujours exact et là encore on obtient des expositions dangereuses pour la santé. En outre, le calcul de la dégressivité de la puissance se base sur la puissance théorique délivrée par l'appareil (exprimée en J/s) et non sur l'éclairement énergétique (exprimé en W/cm2) émis réellement par les tubes à l'intérieur du lit ou de la cabine de bronzage.

Le but de l'invention est de rendre beaucoup plus fiable les appareils de bronzage et de protéger la santé des utilisateurs.

Ce but est atteind à l'aide d'une installation de bronzage comprenant un appareil de bronzage du type comprenant des moyens d'émission d'un rayonnement solaire artificiel destiné à irradier au moins une partie du corps d'un utilisateur au cours d'une séance d'irradiation et des moyens de temporisation de la durée de la séance d'irradiation. Selon les caractéristiques de l'invention, l'installation comprend des moyens d'asservissement du fonctionnement des moyens de temporisation et/ou des moyens d'émission à l'aide de la combinaison :
- de moyens de mesure de l'éclairement énergétique du rayonnement U.V.A émis par les moyens d'émission,
- d'au moins un objet portatif comportant des données propres à l'utilisateur et représentatives de ses capacités de tolérance au rayonnement solaire, et
- d'un récepteur d'au moins l'un desdits objets portatifs destiné à coopérer avec celui-ci.

Il est possible de classer les utilisateurs par exemple selon trois types de peau (ceux à peau claire, ceux à peau normale et ceux à peau mate) ou de façon plus fine selon quatre types (type celtique, type européen à peau claire, type européen à peau brune ou type méditerranéen). Leur type de peau détermine une durée moyenne d'exposition au rayonnement pour une séance et l'évolution de cette durée sur plusieurs séances. Ainsi, un utilisateur à peau mate est susceptible de subir des séances d'exposition plus longues et plus précocement au cours d'une série, qu'un utilisateur à peau claire. A titre d'exemple, un utilisateur à peau mate pourra subir une exposition de 10 minutes dès la deuxième séance, tandis qu'un utilisateur à peau claire ne pourra subir qu'une exposition de 5 minutes et devra attendre la quatrième séance pour rester exposé pendant 10 minutes.

Grâce aux caractéristiques de l'invention, il est possible en connaissant le type de peau de l'utilisateur et l'éclairement énergétique des moyens d'émission d'augmenter ou de diminuer automatiquement la durée d'une séance d'irradiation ou d'allumer ou d'éteindre certains des tubes qui constituent les moyens d'irradiation.

De préférence, l'objet portatif comporte des données concernant la date et l'heure de la dernière séance effectuée par l'utilisateur. Ainsi, pour des raisons de sécurité, l'utilisateur ne pourra pas effectuer deux séances successives de façon trop rapprochée. Il est en effet souhaitable de laisser un intervalle de temps de 24 heures minimum entre deux séances.

L'invention peut également être adaptée à des appareils de bronzage existants et à cet effet concerne aussi un dispositif de régulation d'un appareil de bronzage du type comprenant des moyens d'émission d'un rayonnement solaire artificiel destiné à irradier au moins une partie du corps d'un utilisateur au cours d'une séance d'irradiation, caractérisé en ce qu'il comprend des moyens de temporisation de la durée de la séance d'irradiation, des moyens de régulation de la puissance du rayonnement émis et des moyens d'asservissement du fonctionnement desdits moyens de temporisation et/ou des moyens d'émission à l'aide de la combinaison :
- de moyens de mesure de l'éclairement énergétique du rayonnement U.V.A émis par les moyens d'émission,
- d'au moins un objet portatif comportant des données propres à l'utilisateur et représentatives de ses capacités de tolérance au rayonnement solaire, et
- d'un récepteur d'au moins l'un desdits objets portatifs destiné à coopérer avec celui-ci.

L'invention concerne également un procédé de régulation d'un appareil de bronzage qui selon les caractéristiques de l'invention comprend les étapes consistant à :
- déterminer une durée de base d'une séance d'irradiation en fonction de données propres à l'utilisateur et représentatives de ses capacités de tolérance au rayonnement solaire et en fonction du nombre et/ou de la durée des séances d'irradiation déjà subies par l'utilisateur,
- mesurer l'éclairement énergétique des moyens d'émission, et
- moduler automatiquement la durée de la séance d'irradiation et/ou la puissance émise par les moyens d'émission en fonction de ces données.

L'invention concerne enfin l'objet portatif associé décrit ci-dessus.

L'invention sera mieux comprise à la lecture de la description suivante d'un mode de réalisation de l'invention, donné à titre d'exemple illustratif et non limitatif, cette description étant faîte en faisant référence aux dessins joints dans lesquels :
- la figure 1 est un schéma illustrant la structure de l'installation selon l'invention,
- la figure 2 est un schéma illustrant l'unité centrale de l'ordinateur utilisé dans l'installation selon l'invention, et
- les figures 3 à 5 sont des algorithmes représentant les diverses étapes de fonctionnement de l'installation et de son unité centrale.

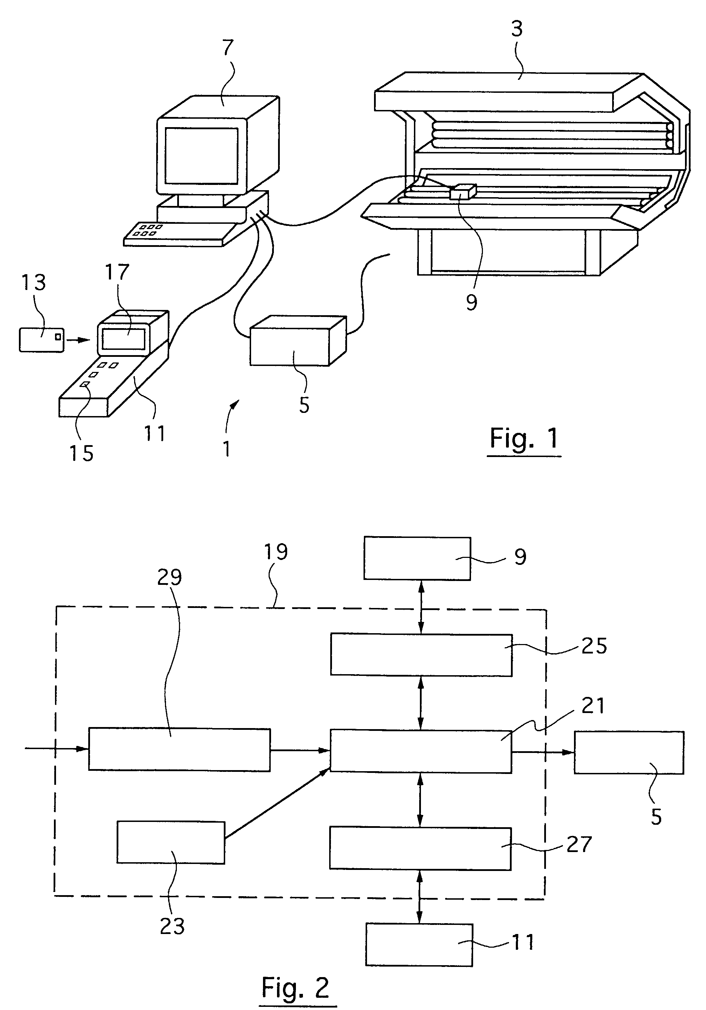

Comme illustré en figure 1, l'installation de bronzage 1 selon l'invention comprend un appareil de bronzage 3 relié à un contacteur de puissance 5 et à un ordinateur 7. Ce dernier est en outre relié à un capteur de mesure 9 de la puissance délivrée par les lampes ou tubes de l'appareil de bronzage et à un lecteur 11 de carte à puce et/ou magnétique 13. Ce lecteur de carte comprend un clavier 15 d'introduction de données et un écran d'affichage 17.

L'appareil de bronzage 3 comprend des moyens d'émission d'un rayonnement solaire artificiel constitué par exemple de tubes fluorescents dits 'basse pression" ou d'émetteurs à vapeur de mercure dits "haute pression".

La carte à puce et/ou magnétique 13 est personnelle à chaque utilisateur de l'appareil de bronzage et contient des informations sur :
- le nombre de séances déjà effectuées par l'utilisateur et/ou le nombre de séances restantes,
- la date et l'heure de la dernière séance,
- le type de sa carnation de peau, et
- d'éventuelles indications médicales dans les cas de maladies de peau telles que le psoriasis ou le vitiligo, par exemple, nécessitant une irradiation aux U.V. (photothérapie).

Le clavier 15 du lecteur de carte permet l'initialisation d'une nouvelle carte 13 et la saisie d'un code autorisant le fonctionnement de l'installation.

L'écran d'affichage 17 à cristaux liquides a pour rôle d'informer l'utilisateur, par l'intermédiaire de messages envoyés par l'ordinateur, de ce qu'il doit faire, de la durée de la séance, du nombre de séances déjà effectuées et/ou du nombre de séances restantes, etc.

Le capteur de mesure 9 est constitué d'au moins une photodiode (ou capteur d'U.V.), placée sur un support et permettant de connaître l'éclairement énergétique sous forme d'U.V.A , en mW/cm2. De façon avantageuse, le support comprend trois capteurs placés respectivement sur le dessus, le dessous et le côté du support afin de mesurer la totalité des U.V. émis dans un lit ou une cabine de bronzage.

L'ordinateur 7 comprend une unité centrale 19 illustrée en figure 2. Cette unité centrale comprend un microprocesseur 21, par exemple un microprocesseur 80C652 commercialisé par la société INTEL qui sert à exécuter le programme et à commander le reste de l'installation. L'unité centrale comprend également une horloge 23. Celle-ci permet à l'unité centrale 19 de connaître la date et l'heure exacte, afin de vérifier que l'intervalle minimum de temps entre deux séances est bien respecté et de calculer la durée exacte d'exposition de chaque séance.

L'unité centrale comprend par ailleurs un convertisseur analogique/numérique 25 permettant de transformer les valeurs lues par le capteur de mesure 9 en données compréhensibles par le microprocesseur 21. L'unité centrale comprend également une interface de liaison 27 avec le lecteur de carte 11 et un dispositif de régulation de la tension et du courant 29 reliant le microprocesseur 21 à l'alimentation électrique. Enfin, le microprocesseur 21 est relié au contacteur de puissance 5.

Le contacteur de puissance 5 permet l'alimentation électrique des lampes à U.V. pendant la durée d'un séance, sous le contrôle du microprocesseur 21 de l'unité centrale.

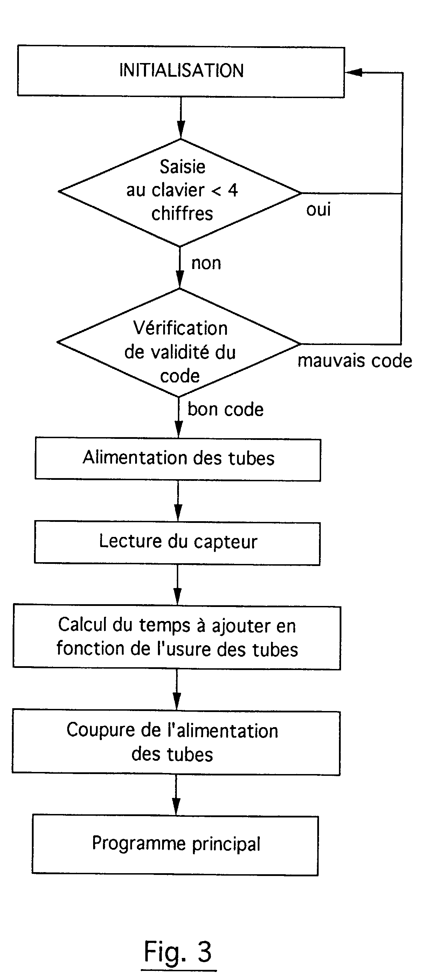

L'algorithme de la figure 3 représente les étapes de mise en marche de l'installation selon l'invention. L'installation est mise sous tension puis est mise en attente jusqu'à la saisie d'un code de mise en marche connu uniquement de la personne qui gère le centre de bronzage. Si le code fait au moins 4 chiffres (par exemple), il est vérifié et s'il est correct, le contacteur de puissance 5 est mise en marche, ce qui a pour effet d'allumer les tubes de l'appareil de bronzage 3. On laisse chauffer les tubes pendant 4 minutes et le capteur 9 effectue une mesure. Ce capteur peut, soit être posé sur le lit ou dans la cabine de bronzage au moment de la mesure qui est alors effectuée de façon ponctuelle, par exemple une fois par jour, soit être installé de façon définitive sur l'appareil de bronzage pour effectuer des mesures en continu. La valeur mesurée est comparée à la valeur d'origine (lorsque le tube est neuf) et l'unité centrale 21 calcule le temps à ajouter aux durées des séances à venir (c'est à dire les séances ayant lieu jusqu'à la prochaine mesure par le capteur). Le temps ajouté est par exemple de 1 minute pour 3 mW/cm2 d'éclairement énergétique perdu, avec un maximum de 5 minutes. Ensuite, le contacteur de puissance 5 est arrêté et l'installation est mise en veille, en attente de l'introduction d'une carte d'un utilisateur ou de la lecture d'un code, (retour au programme principal).

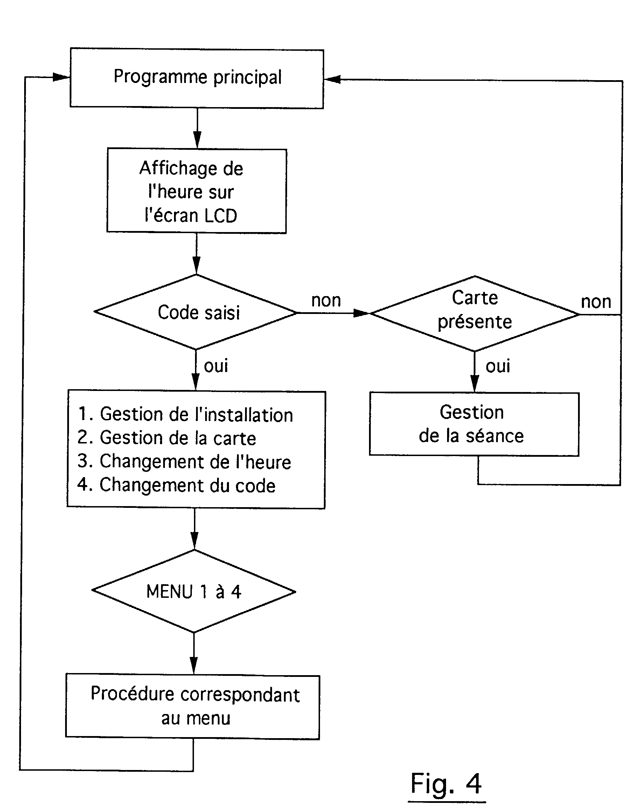

L'algorithme de la figure 4 illustre le programme de scrutation du clavier et d'attente d'une carte. Si aucun code n'est saisi sur le clavier 15 mais qu'une carte 13 est introduite dans le lecteur, la séance peut commencer. Si un code est saisi, le choix de menus possible s'affiche sur l'écran. Ces menus sont les suivants : changement de l'heure, changement du code, gestion du système et gestion de la carte. En fonction du menu choisi, celui-ci est effectué.

Le menu de changement de l'heure permet de saisir une nouvelle date et une nouvelle heure et de mettre à jour le système. Le menu de changement du code permet de saisir un nouveau code (par exemple lors du changement de l'exploitant du centre de bronzage) et de sauvegarder celui-ci. Le menu de gestion de l'installation permet à l'exploitant du centre de bronzage de saisir le nombre de tubes en fonctionnement (20, 30, 40, 50 ou 60 par exemple) et au capteur 5 d'effectuer la mesure de la puissance émise par les tubes. Ensuite, en fonction de l'usure observée, l'unité centrale calcule le temps supplémentaire d'exposition à ajouter au temps initial. Enfin, le menu de gestion d'une carte permet de saisir le type de peau de l'utilisateur, de calculer la durée de base d'une séance en conséquence et le nombre total de séances autorisées et de remettre à zéro la date du dernier passage de l'utilisateur, ainsi que de sauvegarder ces données sur la carte.

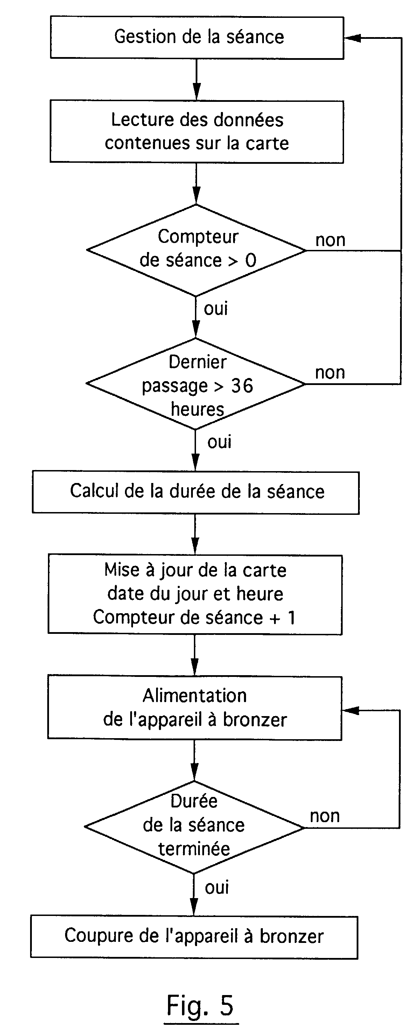

L'algorithme de la figure 5 illustre le programme de gestion de la durée d'une séance. Les données contenues sur la carte de l'utilisateur sont lues et le nombre de séances restantes et la date du dernier passage sont lues et vérifiées. Si tout est correct (c'est à dire qu'il reste au moins une séance et que le dernier passage est antérieur de plus de 36 heures), alors la durée de la séance est calculée et s'affiche sur l'écran. Cette durée est calculée en fonction du nombre de séances déjà subies par l'utilisateur, de son type de carnation, du nombre de tubes de l'installation et de l'usure de ceux-ci. En outre, la carte est mise à jour (c'est à dire qu'une séance est décomptée).

Les tubes de l'appareil de bronzage sont alors alimentés durant la durée calculée comme précédemment, puis l'alimentation de ces tubes est coupée.

## Revendications

1. Installation de bronzage (1) comprenant un appareil de bronzage (3) du type comprenant des moyens d'émission d'un rayonnement solaire artificiel destiné à irradier au moins une partie du corps d'un utilisateur au cours d'une séance d'irradiation et des moyens de temporisation (23) de la durée de la séance d'irradiation, caractérisée en ce qu'elle comprend des moyens d'asservissement du fonctionnement des moyens de temporisation et/ou des moyens d'émission à l'aide de la combinaison :
- de moyens de mesure (9) de l'éclairement énergétique du rayonnement U.V.A émis par les moyens d'émission,
- d'au moins un objet portatif (13) comportant des données propres à l'utilisateur et représentatives de ses capacités de tolérance au rayonnement solaire, et
- d'un récepteur (11) d'au moins l'un desdits objets portatifs destiné à coopérer avec celui-ci.

2. Installation de bronzage selon la revendication 1, caractérisée en ce que l'objet portatif (13) comporte des données concernant le type de peau de l'utilisateur, le nombre total de séances, le nombre de séances déjà écoulées et/ou le nombre de séances restantes.

3. Installation de bronzage selon la revendication 2, caractérisée en ce que l'objet portatif (13) comporte des données concernant la date et l'heure de la dernière séance.

4. Installation de bronzage selon l'une des revendications précédentes, caractérisée en ce que l'objet portatif (13) est une carte magnétique et/ou une carte à puce et en ce que le récepteur (11) est un lecteur de carte.

5. Dispositif de régulation d'un appareil de bronzage (3) du type comprenant des moyens d'émission d'un rayonnement solaire artificiel destiné à irradier au moins une partie du corps d'un utilisateur au cours d'une séance d'irradiation, caractérisé en ce qu'il comprend des moyens de temporisation (23) de la durée de la séance d'irradiation, des moyens de régulation de la puissance du rayonnement émis et des moyens d'asservissement du fonctionnement desdits moyens de temporisation et/ou des moyens d'émission à l'aide de la combinaison :
- de moyens de mesure (9) de l'éclairement énergétique du rayonnement U.V.A émis par les moyens d'émission,
- d'au moins un objet portatif (13) comportant des données propres à l'utilisateur et représentatives de ses capacités de tolérance au rayonnement solaire, et
- d'un récepteur (11) d'au moins l'un desdits objets portatifs destiné à coopérer avec celui-ci.

6. Dispositif de régulation d'un appareil de bronzage (3) selon la revendication 5, caractérisé en ce que l'objet portatif (13) comporte des données concernant le type de peau de l'utilisateur, le nombre total de séances, le nombre de séances déjà écoulées et/ou le nombre de séances restantes.

7. Dispositif de régulation d'un appareil de bronzage (3) selon la revendication 5, caractérisé en ce que l'objet portatif (13) comporte des données concernant la date et l'heure de la dernière séance.

8. Dispositif de régulation d'un appareil de bronzage (3) selon la revendication 5, caractérisé en ce que l'objet portatif (13) est une magnétique et/ou une carte à puce et en ce que le récepteur (11) est un lecteur de carte.

9. Procédé de régulation d'un appareil de bronzage (3) inclut dans une installation selon l'une des revendications 1 à 4 et du type comprenant des moyens d'émission d'un rayonnement solaire artificiel destiné à irradier au moins une partie du corps d'un utilisateur au cours d'une séance d'irradiation, caractérisé en ce qu'il comprend les étapes consistant à :
- déterminer une durée de base d'une séance d'irradiation en fonction de données propres à l'utilisateur et représentatives de ses capacités de tolérance au rayonnement solaire et en fonction du nombre et/ou de la durée des séances d'irradiation déjà subies par l'utilisateur,
- mesurer l'éclairement énergétique des moyens d'émission, et
- moduler la durée de la séance d'irradiation et/ou la puissance émise par les moyens d'émission en fonction de ces données.

10. Objet portatif apte à coopérer avec l'installation ou le dispositif selon l'une des revendications 1 à 8, caractérisé en ce qu'il comprend des moyens de stockage de données concernant le type de peau de l'utilisateur, le nombre total de séances, le nombre de séances déjà écoulées et/ou le nombre de séances restantes et la date et l'heure de la dernière séance.
